# EUROPÄISCHE PATENTANMELDUNG

(11) **EP 1 095 667 A2**
(43) Veröffentlichungstag der Anmeldung: **02.05.2001**
(21) Anmeldenummer: 00123774.2
(22) Anmeldetag: 01.11.2000
(51) Int. Cl.: A61M 5/14, A61C 5/04, A61F 2/24, A61B 17/56

(54) **Vorrichtung zum Einbringen von Knochenzement in ein Knochenrohr**

(30) Priorität: 01.11.1999 DE 29919110 U
(71) Anmelder: Dunsch-Herzberg, Renate, D-22880 Wedel (DE); Voss, Gudrun, D-25491 Hetlingen (DE)
(72) Erfinder: Herzberg, Wolfgang, Dr. med., 22880 Wedel (DE)
(74) Vertreter: Richter, Werdermann & Gerbaulet

(57) **Zusammenfassung**

Bei einer Vorrichtung (100) zum Einbringen von Knochenzement (12) in den Hohlraum eines Knochens (16), wobei der Knochenzement über ein Füllrohr (14) in das Innere des Knochenrohres gebracht wird, weist die Austrittsöffnung (15) des Füllrohres (14) einen trichterförmig ausgebildeten und veränderbaren Querschnitt auf. Dadurch kann sich die Austrittsöffnung (15) der jeweiligen lichten Weite des Knochenrohres anpassen, so dass an der Einfüllstelle ein konstanter Einfülldruck des Knochenzementes aufrecht erhalten wird. Weiterhin ist ein Überwurfrohr (13) vorgesehen, das durch Ausbildung eines Ringspaltes zum Füllrohr (14) eine Absaugeinrichtung für verdrängte Flüssigkeiten, wie zum Beispiel Blut, bildet. Das Überwurfrohr (13) kann ferner durch Verschieben über die Austrittsöffnung (15) hinaus eine Querschnittsbegrenzung der Austrittsöffnung bewirken, so dass die Vorrichtung (100) problemlos in das Knochenrohr des Knochens eingeführt werden kann, wobei das Überwurfrohr (13) an seinem Ende ebenfalls einen veränderbaren Querschnitt aufweist, so dass es sich an die innere Knochenwand anlegt und für eine Konzentration der Absaugwirkung sorgt. Über zusätzliche Spülleitungen kann Spülflüssigkeit zugeführt werden.

## Beschreibung

Die Erfindung betrifft eine Vorrichtung zum Einbringen von Knochenzement in ein Knochenrohr, gemäß dem Oberbegriff des Anspruches 1.

Eine Vorrichtung der eingangs genannten Art ist bekannt und wird dafür verwendet, eine Prothese in ein Knochenrohr zu zementieren. Die Qualität einer solchen Zementierung ist dabei davon abhängig, wie innig der entstehende Verbund von Zement und Knochen wird. Da die Spalträume des Knochengewebes zum Zeitpunkt der Zementierung mit Blut gefüllt sind, hängt die Qualität der Zementierung nicht zuletzt davon ab, inwieweit es gelingt, das Blut aus den Spalträumen zu entfernen und diese gleichzeitig mit Zement zu füllen. Dabei kann der Austausch von Zement gegen Blut nur an der Zement-Füllgrenze vonstatten gehen, da eine spätere Verpressung von Blut nach Abschluss der Zementfüllung kaum noch möglich ist, so wie auch Lufteinschlüsse nach Abschluss der Zementfüllung nicht mehr entfernt werden können.

Die geschilderten Probleme werden mit einer eingangs genannten Vorrichtung zu lösen versucht. Bei einer solchen bekannten Vorrichtung handelt es sich um eine standardisierte Zementspritze mit einem langen zylindrischen Füllrohr, das eine "retrograde" Füllung des Knochenrohres ermöglicht. Mit dieser Technik kann verhindert werden, dass größere Blutdepots an der Zement-Knochen-Grenze ("Interface") verbleiben können, da diese vom Zement aus der Öffnung des Knochenrohres herausgedrückt werden. Es zeigt sich indes, dass diese Verdrängung des Blutes je nach Position des Füllrohres im Knochen beziehungsweise je nach Patient unterschiedlich gut gelingt. Eine unzureichende Entfernung des Blutes führt jedoch wie oben erläutert zu einer verminderten Qualität der Zementfüllung.

Durch die DE-A-32 29 027 ist eine Düse zum Füllen eines Röhrenknochens mit Zement bekannt. Diese Düse zum Einbringen von Knochenzement in den Medullarkanal eines Röhrenknochens mit einem langen Füllrohr, dessen eines Ende an einem Knochenzementgeber anschließbar ist, ist in der Weise ausgebildet, dass am Ausflussende des Füllrohres ein zementundurchlässiger Schirm befestigt ist, der eine biegsame Außenkante aufweist, wobei der Schirm zwischen einer dem Umfang des Füllrohres entsprechenden zusammengeklappten Lage beim Einführen des Füllrohres in den Medullarkanal und einer Spreizlage aufspreizbar ist und in der Spreizlage mit seiner Außenkante eine Abdichtung an der Medullar-Kanalwandung bildet.

Aufgabe der vorliegenden Erfindung war es, eine eingangs genannte Vorrichtung zum Einbringen von Knochenzement in ein Knochenrohr derart zu verbessern, dass über die gesamte Länge eines Knochenrohres hinweg sowie von Patient zu Patient eine gleichmäßig hohe Qualität der Zementfüllung erreicht werden kann. Außerdem soll vermieden werden, dass durch die gegebene Knickspannung des Fächers bei einem Zusammenfalten des Fächers bleibende Verformungen entstehen, so dass es nicht zu Undichtigkeiten im Anlagebereich des entfalteten Fächers an der Innenwandfläche des Knochenrohres kommen kann. Des weiteren soll die Vorrichtung zum Einbringen von Knochenzement auf handelsübliche Zementspritzen aufsetzbar sein, wobei ein zementdichter Abschluss des Füllrohrendes der Zementspritze zum Trägerrohr erreicht werden soll.

Diese Aufgabe wird durch eine Vorrichtung mit den Merkmalen des Anspruches 1 gelöst.

Gemäß einem ersten, in Anspruch 1 beschriebenen Aspekt der Erfindung ist die Vorrichtung zum Einbringen von Knochenzement dadurch gekennzeichnet, dass die Austrittsöffnung des Füllrohres einen während des Füllvorganges veränderbaren Querschnitt aufweist. Das heißt, dass sich die Weite der Austrittsöffnung, an welcher der Zement in das Knochenrohr übertritt, verringern und vergrößern kann. Insbesondere kann er die jeweils maximale lichte Weite des Knochenrohres annehmen, so dass die Austrittsöffnung des Füllrohres ohne Lücke an der Wand des Knochenrohres anliegt.

Des weiteren ist die Vorrichtung zum Einbringen von Knochenzement entweder als integrierter Bestandteil der Zementspritze oder als flexibler Aufsatz für die Zementspritze ausgebildet. Als flexibler Aufsatz ist die erfindungsgemäße Vorrichtung verwendbar für alle handelsüblichen Zementspritzen. Für diese Ausgestaltung weist die Vorrichtung einen ersten Abschnitt, einen zweiten Abschnitt und einen dritten Abschnitt auf, von dem der erste Abschnitt das Füllrohr der Zementspritze umschließt, wobei im zweiten Abschnitt die Querschnitte des Füllrohres der Zementspritzen auf ein konisch zulaufendes Überwurfrohr oder Trägerrohr treffen, in dem sich die Öffnung des Füllrohres gegen die Innenwandfläche des Trägerrohres bzw. des Überwurfrohres zementdicht abschließt und wobei der Außendurchmesser auf kürzere Strecke bevorzugterweise auf 10 mm zurückgeht, so dass ein für kleine Knochenhohlraumquerschnitte ausreichend schlankes Rohr erhalten wird. Der dritte Abschnitt ist über eine längere Strecke in schlanker Form ausgebildet, also mit einem geringeren Querschnitt versehen, so dass das freie Ende der Vorrichtung tief in das sich verjüngende Knochenrohr einschiebbar ist, ohne dass dabei der einen größeren Durchmesser aufweisende zweite Abschnitt zum ersten Abschnitt sperren kann.

Die aus dem Stand der Technik bekannten Füllrohre für Knochenzement haben eine Austrittsöffnung mit konstantem Querschnitt. Da der Querschnitt eines Knochenrohres je nach Position entlang der Achse des Knochens unterschiedlich ist und da die Querschnitte der Knochenrohre von Patient zu Patient verschieden sind, ist ein derartiges Füllrohr dem Knochenrohrquerschnitt nicht immer optimal angepasst. Es hat sich jedoch gezeigt, dass die wirksame Verdrängung von Blut nur über eine gewisse Zementkompression erreicht werden kann. Da letztere wiederum vom Verhältnis der Querschnitte des Knochenrohres und der Austrittsöffnung des Füllrohres abhängig ist, kann mit der bekannten Einfülltechnik keine gleich bleibende Qualität der Zementierung gewährleistet werden. Vielmehr nimmt die Qualität der Zementierung in dem Maße ab, wie der Knochenquerschnitt in Relation zum Durchmesser des Füllrohres zunimmt. Dieses Problem wird bei der erfindungsgemäßen Vorrichtung vermieden, da der Querschnitt der Ausspritzöffnung des Füllrohres variabel ist und sich somit auf einen optimalen Wert einstellen kann. Ein Optimum liegt dabei in der Regel dann vor, wenn der Querschnitt im wesentlichen dem Innenquerschnitt des Knochenrohres entspricht, das heißt, wenn die Austrittsöffnung ohne Lücken an der Innenwand des Knochenrohres anliegt. Dadurch, dass der Querschnitt der Austrittsöffnung beim Zurückziehen des Füllrohres der jeweils vorherrschenden Querschnittsfläche des Knochenrohres angepasst werden kann, kann über die gesamte Länge des Knochens eine gleich bleibend gute Qualität der Zementierung erreicht werden. In gleicher Weise ermöglicht die Vorrichtung eine optimale Anpassung der Querschnittsfläche der Austrittsöffnung an die individuellen Knochenrohrabmessungen verschiedener Patienten.

Für die Ausgestaltung der im Querschnitt veränderbaren Austrittsöffnung stehen verschiedene Möglichkeiten zur Verfügung, von welchen einige in den Unteransprüchen und den Ausführungsbeispielen beschrieben sind. Grundsätzlich sind dabei Realisierungen denkbar, bei denen über eine aktive Steuerung des Anwenders ein bestimmter Querschnitt der Austrittsöffnung eingestellt werden kann. Dies könnte zum Beispiel mit Hilfe eines entlang des Füllrohres verlaufenden Gestänges erreicht werden. Da es in der Regel jedoch allein darauf ankommt, dass die Austrittsöffnung den von der lichten Knochenweite vorgegebenen maximalen Querschnitt annimmt, wird die Austrittsöffnung vorzugsweise so realisiert, dass sie aufgrund elastischer Kräfte bestrebt ist, einen maximalen Querschnitt anzunehmen. Ohne Gegenkräfte nimmt die Austrittsöffnung somit in der Regel eine etwa trichterförmige Gestalt an, wobei sich innerhalb des Knochenrohres die Öffnungsweite des Trichters an die vorgegebenen Grenzen anpasst. Derartige Maßnahmen sind indes nicht unbedingt erforderlich, da beim Einfüllvorgang aufgrund des Zementdruckes die Austrittsöffnung in der Regel von selbst maximal geweitet wird.

Gemäß Anspruch 2 enthält die Vorrichtung vorzugsweise eine Sperreinrichtung, welche im aktivierten (betätigten) Zustand den maximalen Öffnungsquerschnitt der Austrittsöffnung begrenzt. Eine solche Sperreinrichtung ist insbesondere bei der zuletzt beschriebenen Realisierung der Austrittsöffnung sinnvoll, bei welcher diese aufgrund elastischer Kräfte eine Tendenz zur Annahme der größtmöglichen Öffnungsbreite hat. Eine dermaßen aufgeweitete Austrittsöffnung würde das Einführen der Vorrichtung in einen zu befüllenden Knochen erschweren oder gar verhindern. Aus diesem Grunde wird vorteilhafterweise die genannte Sperreinrichtung vorgesehen, mit welcher während des Einführens der Vorrichtung die Öffnungsweite der Austrittsöffnung begrenzt werden kann. Insbesondere sollte die Öffnungsweite auf einen minimal kleinen Querschnitt einstellbar sein, damit die Vorrichtung möglichst problemlos in den Knochen eingeführt werden kann.

Gemäß Anspruch 3 kann die genannte Sperreinrichtung durch ein Überwurfrohr gebildet werden, welches axial verschiebebeweglich relativ zum Füllrohr und koaxial zum Füllrohr angeordnet ist. Vorzugsweise ist das Überwurfrohr um das Füllrohr herum angeordnet, das heißt, dass sein Innendurchmesser größer ist als der Außendurchmesser des Füllrohres. Durch die axiale Verschiebebeweglichkeit des Überwurfrohres kann dieses in Richtung der Austrittsöffnung des Füllrohres nach vorne geschoben werden, so dass es über die Austrittsöffnung hinaussteht. Hierdurch wird die Ausdehnung der Austrittsöffnung begrenzt, indem sie auf den Innendurchmesser des Überwurfrohres zurückgeführt wird. In dieser Position kann die Vorrichtung problemlos in einen Knochen eingeführt werden, da ihr Durchmesser an der Spitze dem festen Durchmesser des Überwurfrohres entspricht. Gegebenenfalls kann zusätzlich die Spitze des Überwurfrohres geeignet gestaltet werden, z. B. mit abgerundeten Kanten. Wenn die Vorrichtung sich an der gewünschten Position in der Tiefe des Knochenrohres befindet, wird das Überwurfrohr zurückgezogen, so dass es die Austrittsöffnung nicht mehr umschließt und diese sich frei bis zur Innenwand des Knochenrohres entfalten kann.

Eine Möglichkeit zur Realisierung der im Querschnitt veränderlichen Austrittsöffnung besteht gemäß Anspruch 4 darin, dass die Austrittsöffnung des Füllrohres ganz oder teilweise aus einem faltbaren, flexiblen Material besteht und dass dieses Material trichterförmig zugeschnitten ist. Ein solches Material kann sich aufgrund seiner Flexibilität dem jeweiligen Querschnitt des Knochenrohres anpassen und sich an die Wände des Knochenrohres anlegen. Vorzugsweise enthält die Austrittsöffnung dabei elastische Elemente wie zum Beispiel radial verlaufende elastische bzw. elastisch befestigte Stege oder Rippen, welche die Austrittsöffnung maximal zu entfalten versuchen. Dies stellt sicher, dass die Austrittsöffnung immer an der Innenwand des Knochenrohres anliegt.

Bei einer alternativen Ausgestaltung der Austrittsöffnung gemäß Anspruch 5 besteht diese aus sich überlappenden und jeweils einseitig am Füllrohr befestigten Lamellen. Eine solche Anordnung nach Art von Blütenblättern erlaubt ebenfalls eine trichterförmige Veränderung des Öffnungsquerschnittes, wobei aufgrund der Überlappungen der Lamellen sichergestellt ist, dass zu keiner Zeit Lücken im Trichtervorhang der Austrittsöffnung entstehen.

Die Aufgabenstellung der vorliegenden Erfindung wird weiterhin durch eine Vorrichtung mit den Merkmalen nach Anspruch 6 gelöst. Diese Vorrichtung enthält eine Absaugeinrichtung, welche eine bei der Austrittsöffnung des Füllrohres angeordnete Absaugöffnung aufweist. Mit einer derartigen Absaugeinrichtung ist es möglich, das durch den eingefüllten Knochenzement verdrängte Blut beziehungsweise andere Flüssigkeiten aus dem Knochenrohr zu entfernen, so dass sie dort der Zementfüllung nicht mehr im Wege stehen. Durch eine Absaugung wird ferner verhindert, dass derartige Flüssigkeitsansammlungen einen Gegendruck zum eingefüllten Zement aufbauen könnten, welcher ein Eindringen des Zementes verhindert. Ebenso wie die oben beschriebene, im Querschnitt veränderliche Austrittsöffnung des Füllrohres trägt die Absaugeinrichtung also dazu bei, dass der Einschluss von Blut innerhalb des Knochenrohres verhindert wird. Auf diese Weise wird gewährleistet, dass der eingebrachte Knochenzement entlang der gesamten axialen Länge des Knochens eine gleich bleibend gute Qualität besitzt.

Die Absaugöffnung der Absaugeinrichtung ist vorzugsweise in der Nähe der Austrittsöffnung des Füllrohres angeordnet, da an dieser Stelle vornehmlich das durch den eindringenden Knochenzement verdrängte Blut anfällt. Dabei ist natürlich konstruktiv darauf zu achten, dass durch die Absaugöffnung nicht unmittelbar der durch das Füllrohr eingebrachte Zement erfasst wird. Aus diesem Grunde wird in der Regel ein gewisser Abstand zwischen den beiden Öffnungen einzuhalten sein beziehungsweise die Wirkungsbereiche dieser Öffnungen werden vorzugsweise durch einen Trennvorhang separiert.

Gemäß Anspruch 7 kann die Absaugeinrichtung durch ein Überwurfrohr gebildet werden, welches koaxial zum Füllrohr und mit Zwischenraum zum Füllrohr angeordnet ist. Das heißt, dass der Innendurchmesser des Überwurfrohres um einen gewissen Betrag größer ist als der Außendurchmesser des Füllrohres, so dass zwischen beiden Rohren ein Ringspalt entsteht. Durch diesen Ringspalt können Flüssigkeiten wie zum Beispiel verdrängtes Blut aus dem Knochenrohr nach außen abgesaugt werden. Eine solche Anordnung hat den Vorteil, dass die Absaugöffnung ringförmig um das Füllrohr angeordnet ist, so dass sie nach allen Seiten hin eine gleichmäßige Saugwirkung ausüben kann. Darüber hinaus können jedoch durch Löcher in der Wand des Überwurfrohres je nach Bedarf an beliebiger Stelle Absaugöffnungen geschaffen werden.

Gemäß Anspruch 8 kann die Absaugöffnung des Überwurfrohres einen während des Füllvorganges veränderbaren Querschnitt haben. Eine solche Querschnittsveränderung erlaubt insbesondere, dass sich die Breite der Absaugöffnung optimal an den zur Verfügung stehenden lichten Knochendurchmesser anpassen kann. Hierdurch wird die Saugwirkung auf die Zement-Knochen-Grenze konzentriert, so dass dort optimal das Blut und andere Flüssigkeiten entfernt werden.

Ferner kann nach Anspruch 9 die genannte Absaugöffnung veränderlichen Querschnittes dadurch realisiert werden, dass sie aus einem faltbaren, flexiblen Material besteht und trichterförmig zugeschnitten ist. Alternativ hierzu kann gemäß Anspruch 10 die Absaugöffnung aus sich überlappenden und jeweils einseitig am Überwurfrohr befestigten Lamellen bestehen. Die genannten Realisierungsmöglichkeiten für einen veränderlichen Rohrquerschnitt entsprechen denen, welche oben für das Füllrohr beschrieben wurden, so dass sie die dort genannten Vorteile gewähren.

Nach Anspruch 11 ist es bevorzugt, die oben erläuterten Lösungsansätze zur Qualitätssteigerung der Knochenzement-Füllung zu kombinieren, um so einen Synergieeffekt zu erreichen. Das heißt, dass die Vorrichtung zum Einbringen von Knochenzement ein Füllrohr mit einer Austrittsöffnung veränderbaren Querschnittes aufweist sowie darüber hinaus eine Absaugeinrichtung mit einer bei der Austrittsöffnung des Füllrohres angeordneten Absaugöffnung. Bei dieser Vorrichtung sorgt der veränderliche Querschnitt des Füllrohres dafür, dass die Austrittsöffnung in jedem Knochenabschnitt optimal an der Wand anliegt und daher der eingebrachte Knochenzement mit dem gesamten Fülldruck im Knochen eingelagertes Blut verdrängt.

Gleichzeitig wird durch die Absaugeinrichtung ein Sog auf das Blut beziehungsweise andere Flüssigkeiten ausgeübt, welcher zu dessen Entfernung beiträgt. Die Querschnittsveränderung der Austrittsöffnung und die Absaugeinrichtung ergänzen sich somit in der Weise, dass die erste Maßnahme den aktiven Verdrängungsdruck erhöht und die zweite Maßnahme mit einem Absaugunterdruck einen Zug auf zu entfernendes Material ausübt.

Gemäß einer anderen Weiterentwicklung der Erfindung nach Anspruch 12 enthält die Vorrichtung zusätzlich eine Spüleinrichtung zum Einbringen von Spülflüssigkeit. Neben dem Füllrohr zum Einbringen des Knochenzementes ist somit eine zweite Zuleitung für die Zufuhr einer geeigneten Spülflüssigkeit vorhanden. Mit einer derartigen Spülflüssigkeit kann wirkungsvoll die Entfernung von störenden Materialien wie zum Beispiel Blut aus dem Knocheninnenraum unterstützt werden, und es können gegebenenfalls ergänzende medizinische Behandlungen (Einleiten von Antibiotika etc.) durchgeführt werden. Die Spüleinrichtung wird vorzugsweise ergänzt durch eine Absaugeinrichtung der oben erläuterten Art, welche die eingebrachte Spülflüssigkeit aus dem Knocheninnenraum wieder entfernt.

Nach einer weiteren Ausgestaltung der Erfindung besteht die Vorrichtung zum Einbringen von Knochenzement in ein Knochenrohr aus einem Füllrohr mit einem einendseitig angeordneten Anschlussstutzen für den Anschluss an die Zementspritze, aus einem das Füllrohr aufnehmenden einendseitig konisch oder zylindrisch ausgebildeten Trägerrohr mit am freien Spritzenkörper abgewandten Ende angeordneten Faltenschirm und aus einem auf das freie Ende des Trägerrohres aufgeschobenen konischen oder zylindrischen Überwurfrohr mit einer Reißleine zum Abziehen des Überwurfrohres vom konischen oder zylindrischen Endabschnitt des Trägerrohres.

Nach einer weiteren Ausführungsform der Erfindung ist das Überwurfrohr als Aufreißhülse ausgebildet. Diese Aufreißhülse kann mit einem Längsschlitz versehen sein, wobei dann aufgrund einer vorgegebenen Eigenspannung der Faltenschirm in geschlossener und zusammengefalteter Stellung zusammengehalten wird.

An seinem dem Faltenschirm zugekehrten Ende weist das Trägerrohr außenwandseitig eine schneidmesserartig ausgebildete Rücklaufsperre zur Verhinderung eines ungewollten Zurückgleitens des Überwurfrohres bzw. der Aufreißhülse beim Einschieben in das Knochenrohr und zum Aufschlitzen bzw. Aufweiten des Überwurfrohres bzw. der Aufreißhülse bei kräftiger Zugeinwirkung auf. Ist die eingesetzte Aufreißhülse mit einem Längsschlitz versehen, dann wird vermittels mit der Rücklaufsperre die Aufreißhülse bei einem Zurückziehen geweitet und kann somit entfernt werden. In dem Fall, bei dem die Aufreißhülse als geschlossener Körper ausgebildet ist, wird bei einem Zurückziehen der Aufreißhülse diese mittels der schneidmesserartig ausgebildeten Rücklaufsperre aufgeschlitzt und kann somit mühelos entfernt werden.

Weitere vorteilhafte Ausgestaltungen der Erfindung gehen aus den Unteransprüchen hervor.

Ausführungsbeispiele der Erfindung werden anhand der Zeichnungen erläutert.

Es zeigen:
- Fig. 1: einen schematischen Querschnitt durch eine Vorrichtung zum Einbringen von Knochenzement,
- Fig. 2: eine erste Ausgestaltung einer Austrittsöffnung mit veränderbarem Querschnitt,
- Fig. 3: eine zweite Ausgestaltung einer Austrittsöffnung mit veränderbarem Querschnitt,
- Fig. 4: eine dritte Ausgestaltung einer Austrittsöffnung mit veränderbarem Querschnitt,
- Fig. 5: eine Vorrichtung mit einer Absaugeinrichtung um das Füllrohr,
- Fig. 6: eine weitere Ausführungsform einer Vorrichtung zum Einbringen von Knochenzement in ein Knochenrohr, die als flexibler Aufsatz für eine Zementspritze ausgebildet ist, in einer Explosionsdarstellung her einzelnen Aufsatzteile,
- Fig. 7: eine schaubildliche Ansicht des flexiblen Aufsatzes vor dem Einführen des mit einer Anschlusskappe für den Anschluss an eine Zementspritze versehenen Füllrohres in das Trägerrohr mit einem einendseitig angeordneten Faltenschirm, der vermittels eines Überwurfrohres bzw. einer Aufreißhülse in zusammengefalteter Stellung gehalten ist,
- Fig. 8: in einer schaubildlichen Ansicht die zusammengebaute Vorrichtung zum Einbringen von Knochenzement in ein Knochenrohr,
- Fig. 9: teils in Ansicht, teils in einem senkrechten Schnitt die Vorrichtung mit dem von dem Überwurfrohr bzw. der Aufreißhülse in zusammengefalteter Stellung gehaltenen Faltenschirm vor dem Einschieben der Vorrichtung in ein Knochenrohr,
- Fig. 10: in einer schaubildlichen Ansicht die in das Knochenrohr eines Knochens eingeschobene Vorrichtung,
- Fig. 11: in einer schaubildlichen Ansicht die in das Knochenrohr eingeführte Vorrichtung mit entfernter Aufreißhülse und entfaltetem Faltenschirm,
- Fig. 12: in einer schaubildlichen Ansicht die Vorrichtung während des Rückführvorganges aus dem Knochenrohr mit weiterer Entfaltung des Faltenschirmes,
- Fig. 13: in einer schaubildlichen Ansicht die Vorrichtung im Endstadium des Herausziehens aus dem Knochenrohr,
- Fig. 14: teils in Ansicht, teils in einem senkrechten Schnitt das Trägerrohr mit endseitig angeordnetem, entfaltetem Faltenschirm und mit aufgeschobener Aufreißhülse,
- Fig. 15: einen Abschnitt des Tragrohres mit aufgeschobener Aufreißhülse mit verdrehsicherer Führung,
- Fig. 16 A: in einer Seitenansicht das den Faltenschirm tragende freie Ende des Trägerrohres mit aufgesetztem Überwurfrohr bzw. Aufreißhülse,
- Fig. 16 B: in Seitenansichten zwei Falten des Faltenschirmes,
- Fig. 17 A: in einer Seitenansicht das Überwurfrohr bzw. die Aufreißhülse mit einer Reißleine, die durch eine Führung an eine Überwurfmutter geführt ist, die das Füllrohr und das Trägerrohr klemmend zusammenhält,
- Fig. 17 B: eine Vorderansicht auf die Überwurfmutter mit radial angeordneten Führungsaugen für die Reißleine der Aufreißhülse,
- Fig. 17 C: eine Vorderansicht eines Abschnittes der Führung an der Überwurfmutter in einer weiteren Ausgestaltung des Führungsauges für die Reißleine der Aufreißhülse,
- Fig. 18 A: in einer schematischen Seitenansicht das den Faltenschirm tragende Ende des Trägerrohres, wobei der Faltenschirm aus einer Anzahl von Speichen mit zwischen den Speichen angeordneten schwimmhautartigen Folienabschnitten besteht,
- Fig. 18 B: mit einer schematischen Seitenansicht das den Faltenschirm tragende Ende des Trägerrohres mit aufgeschobenem den Faltenschirm zusammenhaltenden Überwurfrohr,
- Fig. 18 C: einen senkrechten Querschnitt gemäß Linie A - A in Fig. 18 B und
- Fig. 19: in einer schematischen Ansicht die in drei Abschnitte mit unterschiedlichen Gestaltungsformen aufgeteilte Vorrichtung zum Einbringen von Zement in ein Knochenrohr.

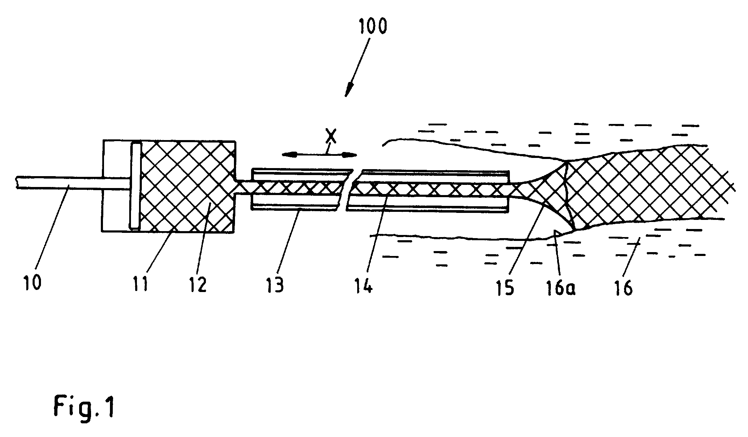

In Fig. 1 ist eine Vorrichtung 100 zum Einbringen von Knochenzement 12 in das Knochenrohr 16a eines Knochens 16 schematisch im Querschnitt dargestellt. Die Vorrichtung enthält im wesentlichen ein lang gestrecktes zylindrisches Füllrohr 14, welches von außen in das Innere des Knochenrohres eingeführt wird. An seinem freiliegenden äußeren Ende ist mit dem Füllrohr 14 die Vorrichtung 100 zum Einbringen des Knochenzementes 12 angebracht, bei der es sich zum Beispiel um einen Spritzenkörper 11 mit einem Kolben 10 einer Zementspritze 110 handeln kann. Durch Druck auf den Kolben 10 wird der Knochenzement 12 durch das Füllrohr 14 und die Austrittsöffnung 15 des Füllrohres in das Innere des Knochenrohres 16a gedrückt.

Bei herkömmlichen Vorrichtungen ist dabei zu beobachten, dass die Qualität der Zementfüllung entlang der Achse des Knochenrohres 16a unterschiedlich gut ausfällt sowie von Patient zu Patient variiert. Ein Grund hierfür ist darin zu suchen, dass im Knochenrohr befindliches Blut unzureichend verdrängt worden ist und sich daher mit dem Knochenzement vermischt und dessen Stabilität herabsetzt.

Eine derartige unzureichende Verdrängung des Blutes beziehungsweise anderer Flüssigkeiten aus dem Knochenrohr wird erfindungsgemäß dadurch vermieden, dass die Austrittsöffnung 15 des Füllrohres 14 einen veränderbaren Querschnitt aufweist. Das heißt, dass sich die Austrittsöffnung 15 trichterartig bis zu einer maximalen Größe erweitert, wobei die maximale Breite durch den Innendurchmesser des Knochenrohres 16a an der jeweiligen Stelle der Austrittsöffnung vorgegeben wird. Eine derartige, im Querschnitt veränderbare Austrittsöffnung 15 legt sich also an jeder Stelle des Knochenrohres 16a dicht an die Knochenwand an und verhindert somit, dass eingefüllter Knochenzement am Füllrohr 14 vorbei im Knochenrohr 16a aufsteigen kann. Der Einfülldruck des Knochenzementes wird dadurch ohne Verluste auf den Bereich der Zement-Knochen-Grenze konzentriert.

So kann während des gesamten Füllvorganges ein konstanter Fülldruck zirkular an der Füllgrenze erzeugt werden und ein gleich bleibend wirksamer Austausch von Blut gegen Zement stattfinden. Diese Konstanz des Fülldruckes lässt sich sowohl über verschiedene Individuen als auch über verschiedene Füllungstiefen (bezogen auf die konische Form des Knochenhohlraumes) gleicher Individuen aufrecht erhalten.

Die Spitze der Vorrichtung ist so gestaltet, dass sie zunächst das Kleinstkaliber des Füllrohres nicht überragt und somit das Einschieben des Rohres in den engen Knochenschaft nicht behindert. Nach Lösen eines sperrenden Mechanismus kann sich die Spitze des Füllrohres 14 öffnen und zirkulären Kontakt zur Innenauskleidung des Knochenrohres 16a herstellen. Der Fülldruck des Zementes sorgt dabei für einen wandständigen Kontakt der sich entfaltenden Rohröffnung. Mit zunehmender Zementfüllung des Knochenrohres 16a schiebt der Füllungsdruck das Füllrohr aus dem Knochenschaft heraus. Gleichzeitig sorgt der Druck für eine bleibende Wandständigkeit der Einfüllöffnung bei stetig zunehmender lichter Knochenrohrweite.

Die Verpackung der gefalteten Spitzen des Füllrohres kann zum Beispiel durch ein Überwurfrohr, einen Aufrissmechanismus oder eine Schnürung erfolgen. Beispielhaft ist aus Fig. 1 ein Überwurfrohr bzw. ein Überwurfrohr 13 erkennbar, welches um das Füllrohr 14 herum angeordnet ist und welches entlang dessen Achse in Pfeilrichtung x vor und zurück bewegt werden kann. Durch Vorschieben des Überwurfrohres 13 über den Bereich der Austrittsöffnung 15 hinaus wird erreicht, dass die Eintrittsöffnung auf den Innendurchmesser des Überwurfrohres 13 zusammengedrückt wird. Das heißt, dass die Spitze der gesamten Vorrichtung vom Überwurfrohr 13 gebildet wird, so dass die Vorrichtung problemlos und ohne anzustoßen in das Innere des Knochenrohres eingeführt werden kann.

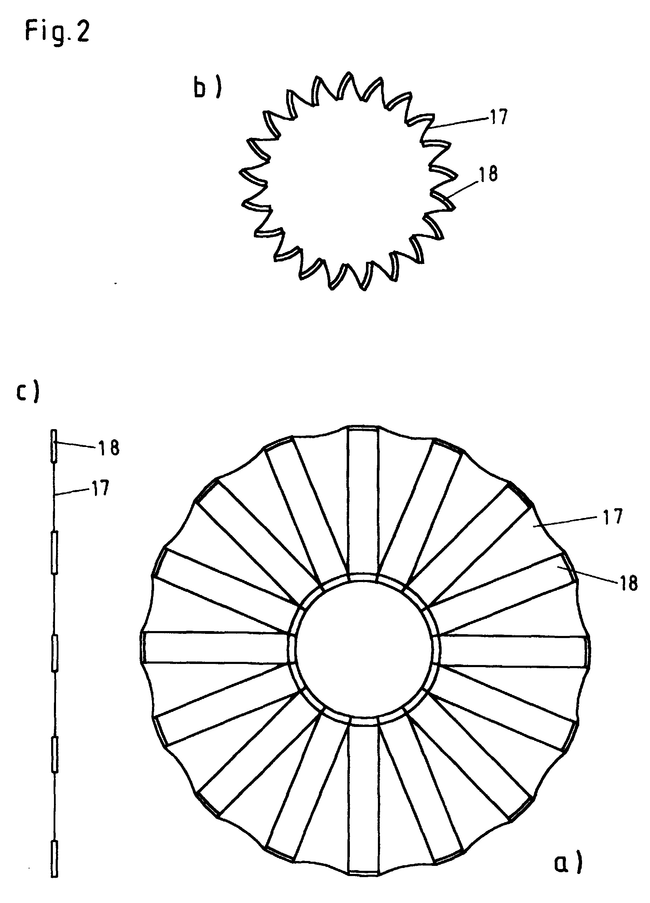

In Fig. 2 ist in drei verschiedenen Ansichten eine spezielle Ausgestaltungsmöglichkeit für die Austrittsöffnung 15 veränderbaren Querschnitts dargestellt. Hierbei handelt es sich um eine Realisierung mit radial von dem Füllrohr 14 abstehenden Streben 18, zwischen denen eine flexible Bespannung 17 ("Schwimmhaut") angeordnet ist. Durch das flexible Material 17 ist es möglich, die Stege 18 relativ zum Füllrohr 14 abzuklappen und die Austrittsöffnung somit nach Art eines Schirmes zu entfalten. In Abb. 2a ist dabei eine Aufsicht auf die voll entfaltete Rosette der Austrittsöffnung 15 dargestellt. Fig. 2b zeigt dieselbe Rosette im fast vollständig zusammengefalteten Zustand, während Fig. 2c eine Seitenansicht auf die vollständig entfaltete Rosette ist.

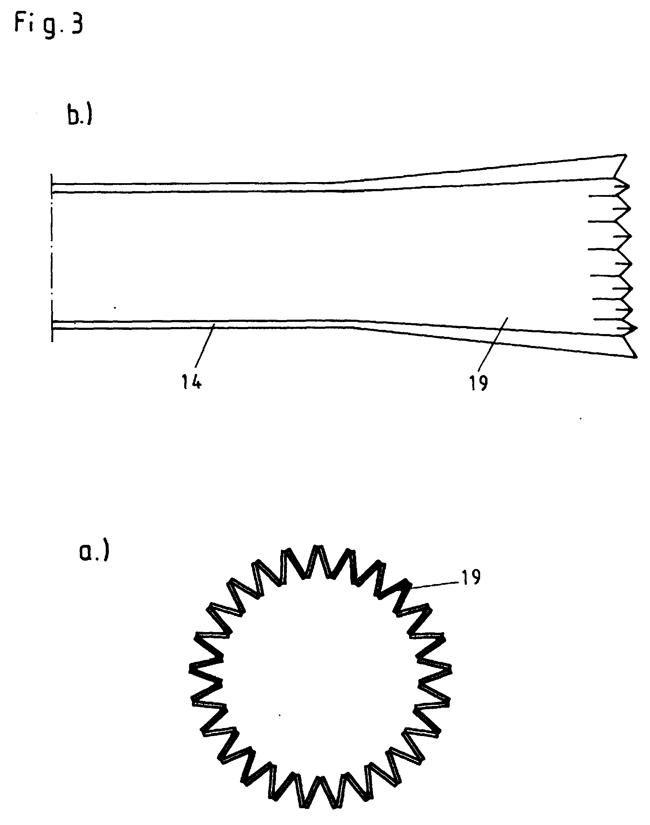

Eine alternative Ausgestaltungsmöglichkeit der im Querschnitt veränderbaren Austrittsöffnung 15 ist in Fig. 3 dargestellt. Hierbei wird die Austrittsöffnung durch ein flexibles Material gebildet, welches trichterförmig zugeschnitten ist und nach Art eines Faltenrockes zickzackförmig zu einem Faltenschirm 150 gefaltet ist, so dass sich die Falten zu einem engeren oder weiteren Querschnitt legen können. Fig. 3a zeigt dabei eine Aufsicht auf die fast vollständig zusammengefaltete Rosette 19, während Fig. 3b eine Seitenansicht beziehungsweise einen Längsschnitt durch das Ende des Füllrohres 14 bei teilweise entfalteter Austrittsöffnung 15 zeigt.

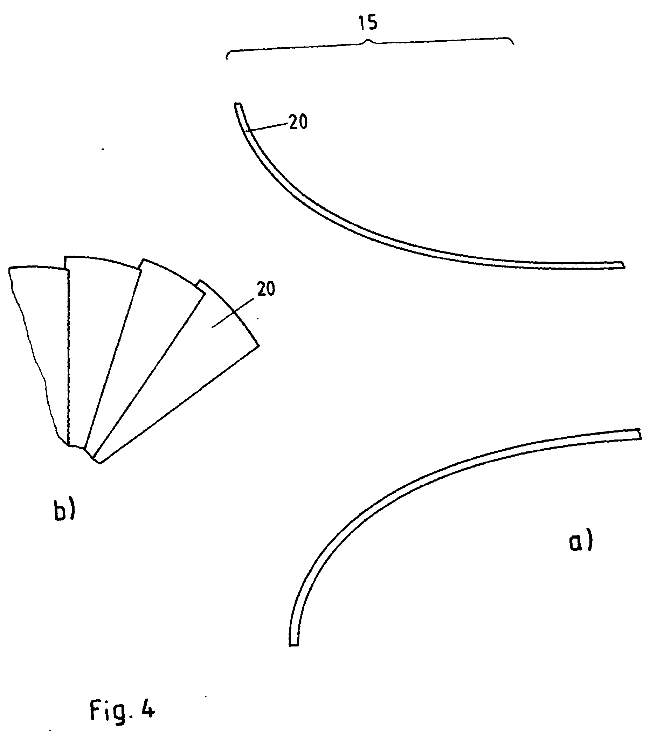

In Fig. 4 ist eine weitere Gestaltungsmöglichkeit für die Austrittsöffnung 15 dargestellt. In diesem Falle wird die Austrittsöffnung durch einzelne Lamellen 20 gebildet, welche sich fächerartig teilweise überlappen und welche an einem Ende an der Öffnung des Füllrohres 14 befestigt sind. Eine Austrittsöffnung aus derartigen Lamellen kann trichterartig verschieden stark geweitet werden, da die einzelnen Lamellen 20 sich relativ zueinander unabhängig bewegen können, wobei ihr teilweiser Überlapp dafür sorgt, dass auch bei größtmöglicher Öffnung von allen Lamellen gemeinsam eine geschlossene Fläche gebildet wird, die den Durchtritt von Knochenzement verhindert. Fig. 4a zeigt einen Längsschnitt durch das Ende des Füllrohres 14 bei teilweise entfalteter Austrittsöffnung 15 und Fig. 4b einen Ausschnitt auf die Aufsicht auf den entfalteten Lamellenfächer. Typischerweise wird die Austrittsöffnung 15 von etwa 12 Lamellen gebildet.

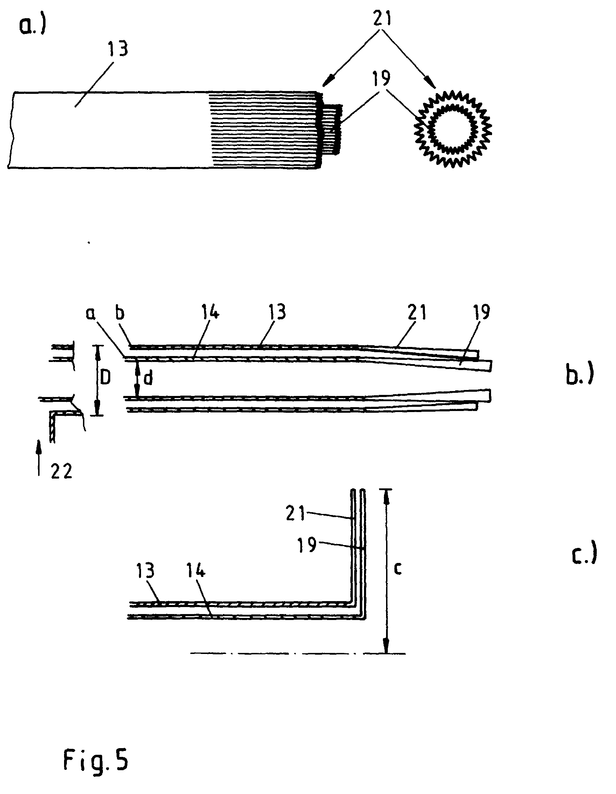

In Fig. 5 ist in verschiedenen Ansichten eine Weiterentwicklung der Erfindung beziehungsweise eine unabhängige Lösungsvariante dargestellt. Diese wird am deutlichsten aus dem Längsschnitt in Fig. 5b erkennbar.

Die Weiterentwicklung besteht darin, dass eine Absaugeinrichtung vorhanden ist, mit der flüssiges Material wie zum Beispiel Blut aus dem Inneren des Knochenhohlraumes entfernt werden kann.

Die Absaugeinrichtung wird in der Ausgestaltung gemäß Fig. 5 dadurch gebildet, dass um das Füllrohr 14 herum ein Überwurfrohr 13 angeordnet ist, wobei der Innendurchmesser des Überwurfrohres um einen gewissen Betrag größer ist als der Außendurchmesser des Füllrohres 14. Hierdurch kommt es zur Ausbildung eines Ringspaltes zwischen den beiden Rohren, durch welchen Flüssigkeit abgesaugt (gegebenenfalls auch eingeführt) werden kann. Die Flüssigkeit wird am Kopf der Vorrichtung über einen in der Figur angedeuteten Absaugstutzen 22 entfernt. Das Absaugen von Flüssigkeit wie zum Beispiel Blut hat den Vorteil, dass diese störenden Materialien vollständig aus dem Knochenhohlraum entfernt werden und somit eine Zementfüllung hoher Qualität entstehen kann.

Wie aus Fig. 5 weiterhin erkennbar ist, werden vorzugsweise sowohl die Austrittsöffnung 15 des Füllrohres 14 als auch die Absaugöffnung des Überwurfrohres 13 in ihrem Querschnitt veränderbar gestaltet. Im dargestellten Beispiel geschieht dies dadurch, dass diese Enden als Faltentrichter 19 beziehungsweise 21 nach Art von Fig. 3 ausgebildet sind. Die Faltenstruktur der Enden wird in der Seitenansicht und in der Aufsicht von Fig. 5a besonders deutlich erkennbar. Der Faltentrichter 19 bewirkt in der oben beschriebenen Weise bei seiner Ausdehnung bis an die Innenwand des Knochenrohres, dass der eingefüllte Zement mit konstantem Fülldruck aus dem Füllrohr 14 austritt. Gleichzeitig trennt das Faltenmaterial 19 den eingefüllten Zement von der Absaugöffnung der Absaugeinrichtung 13. Es ist also ausgeschlossen, dass gerade aus dem Füllrohr ausgetretener Zement unerwünschterweise sofort von der Absaugeinrichtung erfasst und wieder entfernt wird. Die Wirkung der Absaugeinrichtung konzentriert sich vielmehr auf das zu entfernende Material, also insbesondere auf Blutvolumina. Der für das Überwurfrohr 13 eigens vorgesehene Faltentrichter 21 sorgt dabei wiederum dafür, dass sich die Absaugeinrichtung dicht an die Innenwand des Knochenrohres anlegt und die Absaugwirkung somit auf den gerade bearbeiteten Bereich an der Zement-Knochen-Grenze konzentriert wird.

Typische Abmessungen einer Vorrichtung zum Einbringen von Knochenzement betragen mit den in Fig. 5 eingetragenen Bezugszeichen: Außendurchmesser D des Überwurfrohres 13 ca. 12 mm; Innendurchmesser d des Füllrohres 14 ca. 6 mm; Wanddicke a des Füllrohres 14 beziehungsweise Wanddicke b des Überwurfrohres 13 ca. 0,5 mm. Hieraus kann berechnet werden, dass der Abstand zwischen Außenwand des Füllrohres 14 und Innenwand des Überwurfrohres 13 ca. 2,5 mm beträgt. In Fig. 5c ist die von den Faltmaterialien 19 und 21 gebildete Rosette im maximal ausgedehnten Zustand dargestellt, in welchem sie etwa senkrecht von der Wand des Füllrohres 14 beziehungsweise Überwurfrohres 13 absteht. In diesem Zustand beträgt die maximale Öffnungsweite c der Rosette typischerweise ca. 2,5 cm (Radius des Rosettenkreises).

Die in den Figuren dargestellten Ausgestaltungsmöglichkeiten der Erfindung können selbstverständlich miteinander kombiniert werden. So kann bei der Anordnung gemäß Fig. 5 zum Beispiel ein weiteres Überwurfrohr vorgesehen werden, welches durch Vorschieben die Faltentrichter 19 und 21 gebündelt hält und somit eine Einführung der Vorrichtung erleichtert. Weiterhin können Spüleinrichtungen vorgesehen werden, mit welchen gezielt und separat vom Knochenzement Spülflüssigkeit in das Innere des Knochenhohlraumes eingeführt werden können. Derartige Spülflüssigkeiten können dann über die Absaugeinrichtung effizient wieder aus dem Knochenhohlraum entfernt werden. Die während des Zementierungsvorganges durchgeführte Spülung des Knochenrohres (zum Beispiel "Jet-Lavage") führt zusammen mit der gleichzeitigen Absaugung zu einer weiteren Verbesserung der Zementierung.

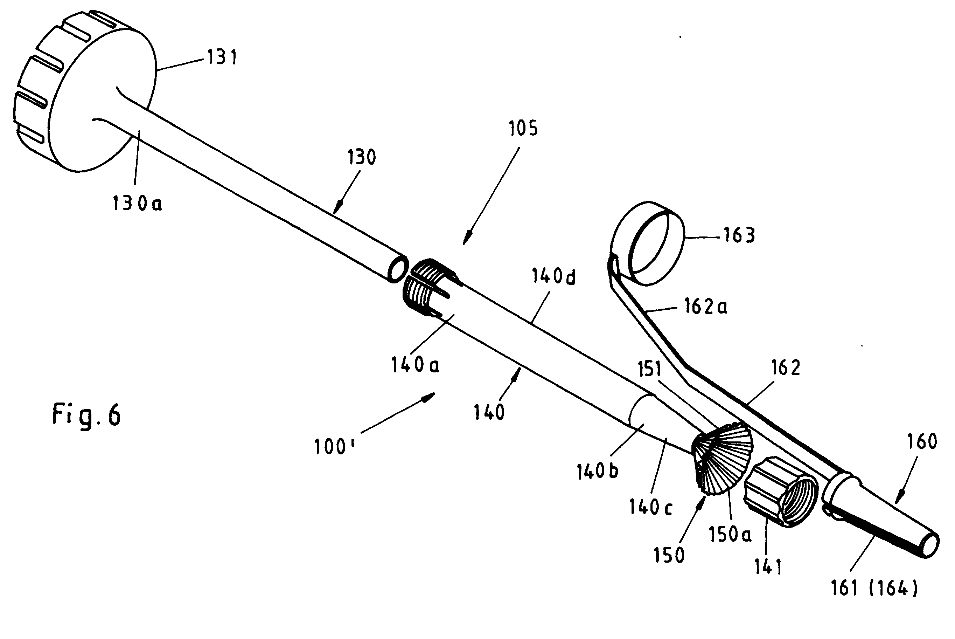

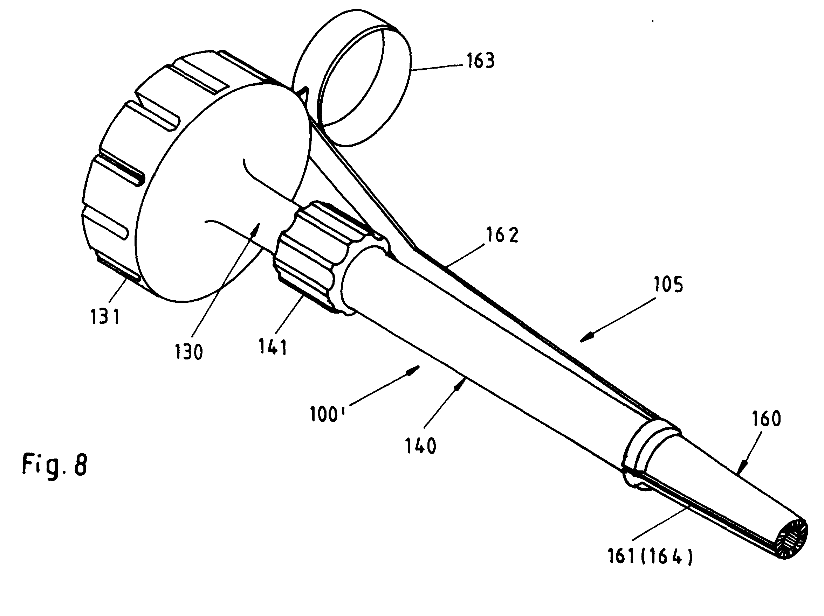

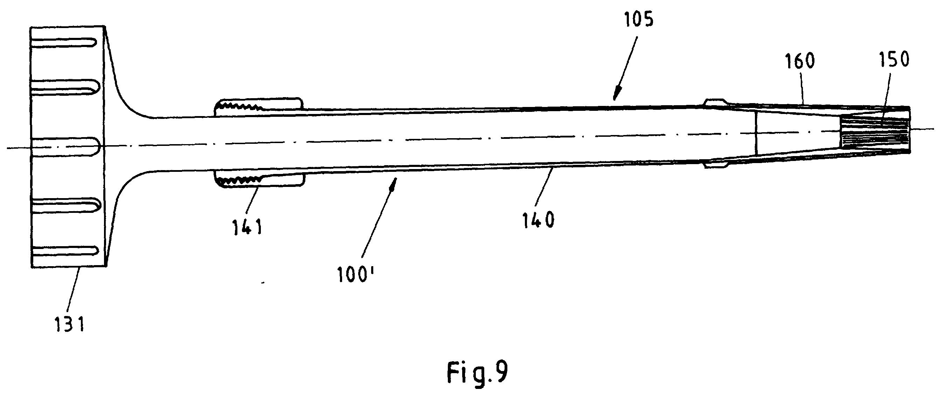

Die Fig. 1 bis 13 zeigen eine weitere Ausführungsform einer Vorrichtung 100' zum Einbringen von Knochenzement in ein Knochenrohr 16a eines Knochens 16. Diese Vorrichtung 100' ist als flexibler Aufsatz 105 für handelsübliche Zementspritzen ausgebildet. Diese Vorrichtung 100' besteht aus einem Füllrohr 130 mit an seinem spritzenseitig zugekehrten Ende 130a vorgesehenen Anschlussstutzen 131 für den Anschluss des Aufsatzes 105 auf eine handelsübliche Zementspritze 110, wobei dieser Anschlussstutzen 131 kappenförmig ausgebildet ist und nach Aufschieben auf den Spritzenkörper an diesem klemmend gehalten ist, wobei auch andere Verbindungsmöglichkeiten eingesetzt werden können. Die Vorrichtung 100' umfasst ferner ein das Füllrohr 130 aufnehmendes Trägerrohr 140, das an seinem spritzenseitig zugekehrten Ende 140a mit einem Gewinde zur Aufnahme einer Feststellmutter 141 versehen ist, vermittels der das Trägerrohr 140 auf dem Füllrohr 130 klemmend gehalten ist (Fig. 8 und Fig. 9). Das Trägerrohr 140 ist zu seinem anderen Ende 140b konisch verjüngend ausgebildet. Der konische Abschnitt ist mit 140c bezeichnet (Fig. 6). An seinem Ende 140b weist das Trägerrohr 140 den vorangehend beschriebenen Faltenschirm 150 auf, der in Abdichtstellung fächerartig bzw. schirmartig auseinander faltbar ist.

Die Vorrichtung 100' umfasst ferner ein Überwurfrohr 13 oder bevorzugterweise bei der in Fig. 1 dargestellten Ausführungsform eine Aufreißhülse 160, die entsprechend dem konischen Abschnitt 140c des Trägerrohres 140 eine entsprechende konische Ausgestaltungen aufweist. Diese konische Aufreißhülse 160 kann auch mit einem Längsschlitz 161 versehen sein. Fig. 6 zeigt die Vorrichtung 100' mit ihren einzelnen Teilen, nämlich dem Füllrohr 130, dem Trägerrohr 140 mit dem Faltenschirm und der Aufreißhülse 160.

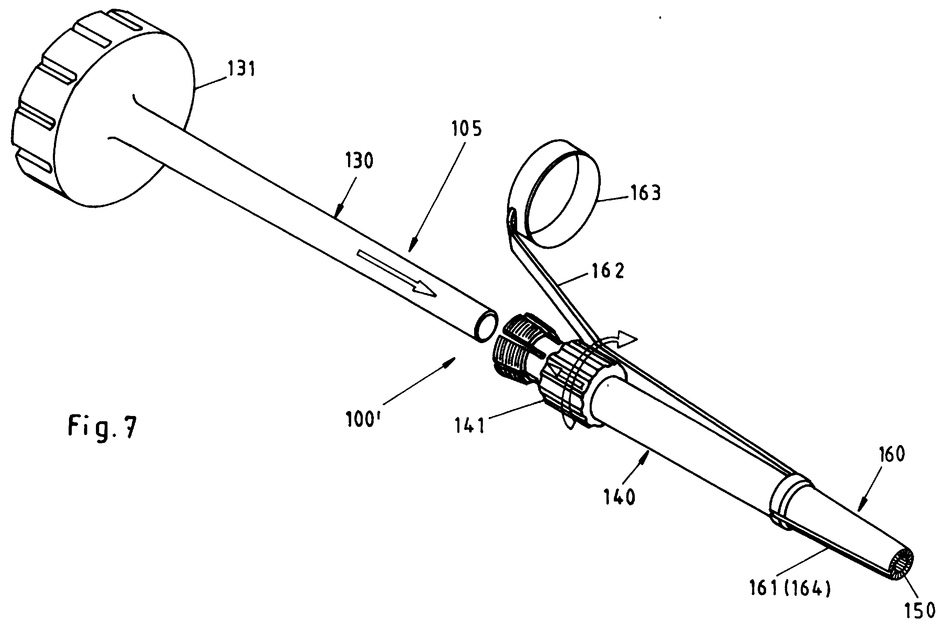

Die Aufreißhülse 160 ist auf dem konischen Abschnitt 140c am Ende 140b des Trägerrohres 140 aufgeschoben und presst dabei den Faltenschirm 150 zusammen (Fig. 7, Fig. 8 und Fig. 9).

Um den Faltenschirm 150 entfalten zu können und um den Faltenschirm in die in Fig. 6 gezeigten Stellung überführen zu können, ist ein Entfernen der Aufreißhülse 160 erforderlich. Hierzu ist die Aufreißhülse 160 mit einer Reißleine 162 versehen, die an ihrem freien Ende 162a mit einer fingerringartigen Handhabe bzw. Lasche 163 versehen ist.

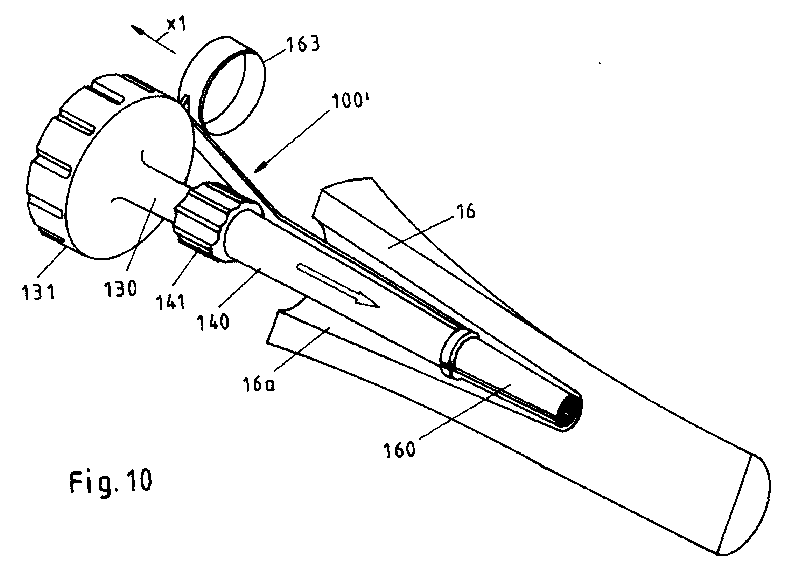

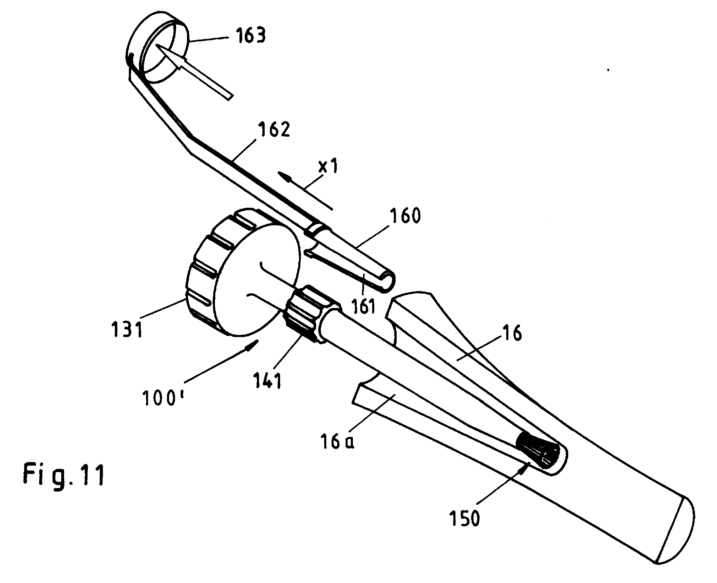

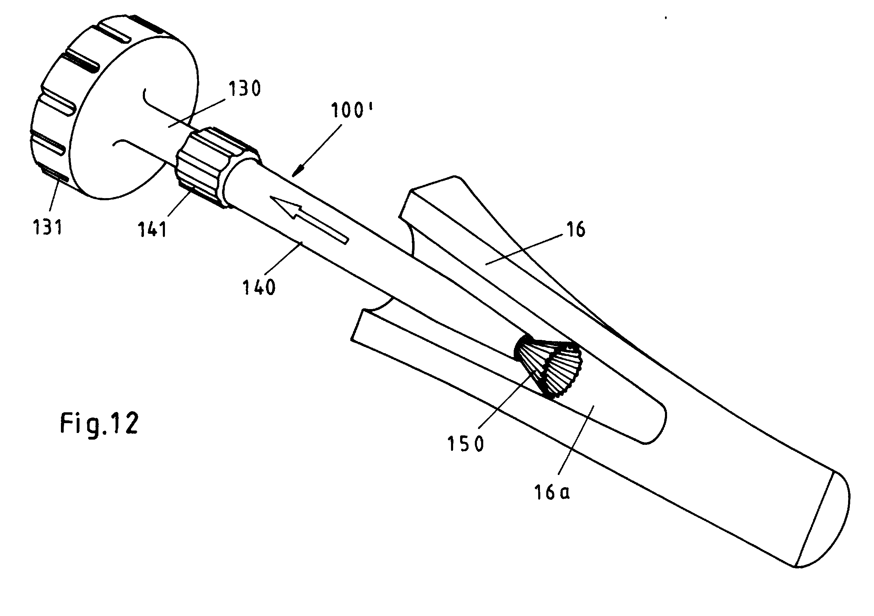

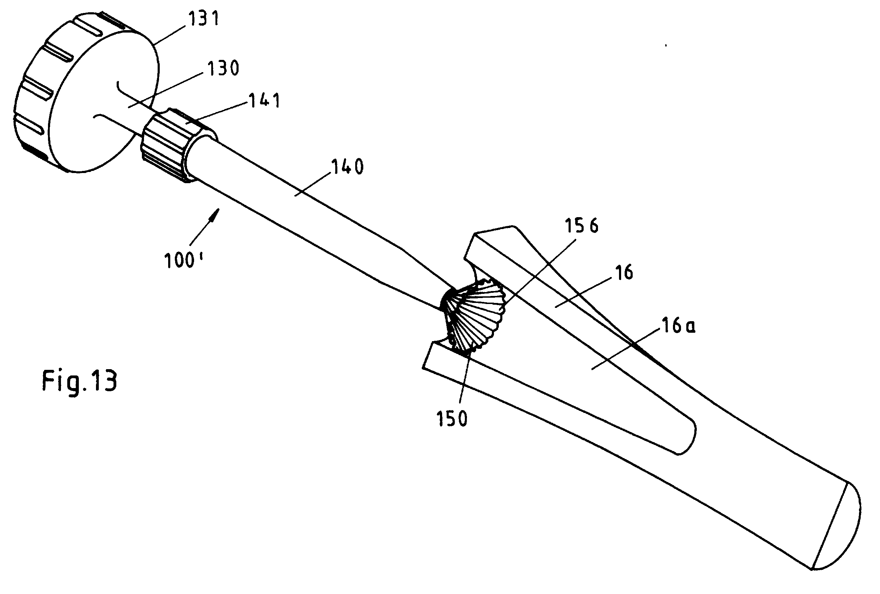

Nach dem Einführen der Vorrichtung 100' in das Knochenrohr 16a des Knochens 16 (Fig. 10) muss der Faltenschirm 150 freigegeben werden, um sich entfalten zu können. Hierzu ist es erforderlich, die Aufreißhülse 160 zu entfernen. Ist die Aufreißhülse 160 mit einem Längsschlitz 161 versehen, dann wird durch ruckartiges Ziehen in Pfeilrichtung x1 die Aufreißhülse 160 über das Trägerrohr 140 abgezogen, wobei sich die Aufreißhülse 160 durch Weiten der den Längsschlitz 161 begrenzenden Schenkel so weit weitet, dass die Aufreißhülse 160 über den sich an den konischen Abschnitt 140c des Trägerrohres 140 anschließenden dickeren Abschnitt 140d des Trägerrohres 140 gezogen werden kann. Die Aufreißhülse 160 öffnet sich dabei und kann somit entfernt werden (Fig. 11). Der Faltenschirm 150 beginnt sich zu entfalten und beim Herausziehen der Vorrichtung 100' aus dem Knochenrohr 16a des Knochens 16 entsprechend Fig. 12 und Fig. 13 entfaltet sich der Faltenschirm 150 fortlaufend und zwar bei gleichzeitiger Anlage des Randes des entfalteten Faltenschirmes 150 an die Innenwand des Knochenrohres 16a des Knochens 16 bis die in Fig. 13 gezeigte Stellung erreicht wird. Der Grad der Entfaltung des Faltenschirmes 150 hängt von den Abmessungen der jeweiligen Konizität des Knochenrohres 16a ab.

Eine Aufreißhülse 160 mit einem Längsschlitz 161 ist bevorzugterweise gefertigt aus einem geeigneten federnd elastischem Material, um eine Eigenspannung zu erhalten, die ausreicht, um den Faltenschirm 150 in geschlossener und zusammengefalteter Stellung zu halten.

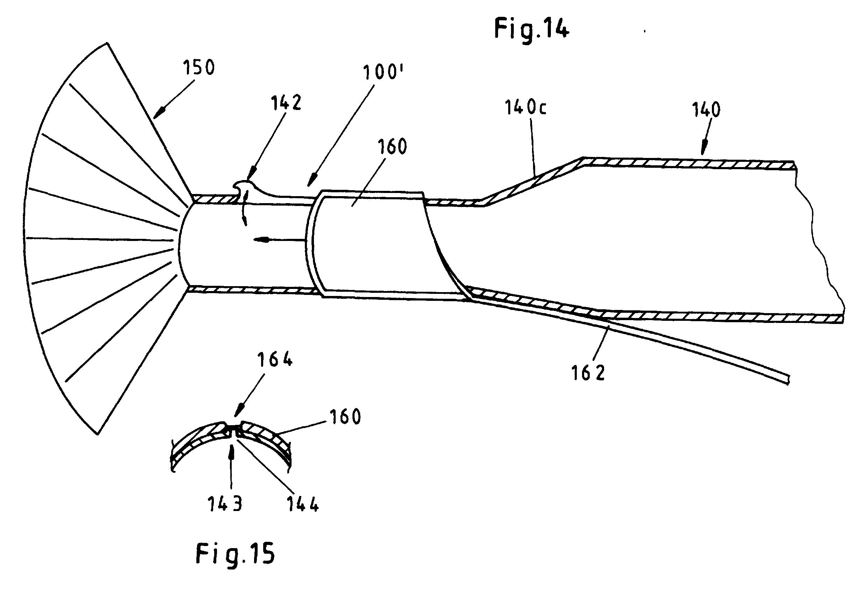

Nach Fig. 14 weist das Trägerrohr 140 an seinem den Faltenschirm 150 tragenden Ende 140b außenwandseitig eine schneidmesserartig ausgebildete Rücklaufsperre 142 auf, vermittels der ein ungewolltes Zurückgleiten der Aufreißhülse 160 beim Einschieben in das Knochenrohr 16a verhindert wird. Außerdem dient die schneidmesserartig ausgebildete Rücklaufsperre 142 zum Aufschlitzen bzw. Aufweiten der Aufreißhülse 160, wenn diese vom Trägerrohr 140 abgezogen werden soll. Weist die Aufreißhülse 160 einen Längsschlitz 161 auf, dann wird dieser Längsschlitz 161 beim Abziehen und beim Vorbeilaufen an der Rücklaufsperre 142 geweitet und kann somit mühelos von dem Trägerrohr 140 abgezogen werden. Weist die Aufreißhülse 160 jedoch keinen Längsschlitz 161 auf, dann wird vermittels der schneidmesserartig ausgebildeten Rücklaufsperre die Aufreißhülse 160 beim Abziehen von dem Trägerrohr 140 aufgeschlitzt. Hierzu bedarf es einer kräftigen Zugeinwirkung. Um das Aufschlitzen der Aufreißhülse 160 bzw. des Überwurfrohres 13 zu bewirken, wenn dieses anstelle der Aufreißhülse 160 eingesetzt wird, ist die Aufreißhülse 160 bzw. das Überwurfrohr 13 mit einer in Längsrichtung verlaufenden Sollbruchstelle 164 versehen (Fig. 15).

Um die Aufreißhülse 160 verdrehsicher auf dem Trägerrohr 140 zu halten und beim Abziehen zu führen, weist das Trägerrohr 140 auf seiner Außenwandfläche einen stabförmigen Längsnocken 143 auf, wohingegen die Aufreißhülse 160 in ihrer Wand mit einer Längsnut 144 versehen ist. Der Längsnocken 143 greift in die Längsnut 144 ein, so dass die Aufreißhülse 160 verdrehsicher auf dem Trägerrohr 140 gehalten und beim Abziehen geführt wird.

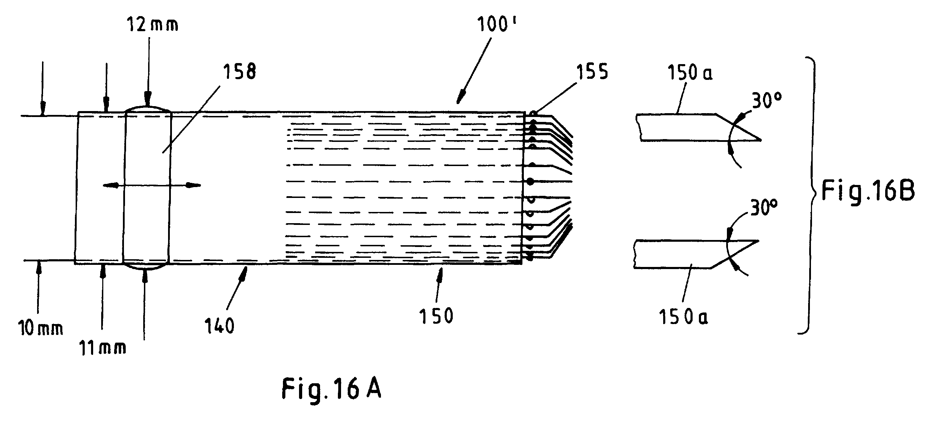

Das Konzept der konischen Schirmkappe, die zur Entsicherung aus dem Knochenrohr 16a des Knochens 16 herausgezogen werden muss, birgt die Gefahr der Eigenverklemmung. Die Spitze kann darum eine zylindrische Form erhalten, auf der ein zylindrisches Überwurfrohr 13 gleiten kann. Dieses Überwurfrohr 13 wird zur Entsicherung nur zurückgezogen und kann nicht verklemmen. Damit dieses Überwurfrohr 13 beim Vorschieben in das Knochenrohr 16a nicht abgestreift werden kann, erhalten die Schirmfalten 151 des Faltenschirmes 150 nahe der Peripherie winzige Noppen 155, die für das Überwurfrohr 13 einen Anschlag bilden und vor Knochenkontakt beim Vorschieben schützt. Diese Noppen 155 sollen soweit vom freien Schirmrand entfernt sein, dass sie beim Entfalten des Faltenschirmes 150 möglichst früh den Knochenkontakt verlieren. Die Gefahr der mangelnden Abdichtung beim Einspritzen in einer frühen Phase der Entfaltung wird somit vermieden. Um eine optimale Dichtung des Faltenrockes des Faltenschirmes 150 gegen den Knochen zu gewährleisten, werden die Falten 150a angeschrägt, so dass sie bei einem Öffnungswinkel von 30° anliegen, also 60° gegen die Faltenlängsachse angeschnitten sind (Fig. 16 A, Fig. 16 B). Das Anschneiden der Falten 150a erleichtert zudem auch das Hineingleiten der Vorrichtung 100' in das Knochenrohr 16a des Knochens 16 und zwar entsprechend einer kufenartigen Wirkung.

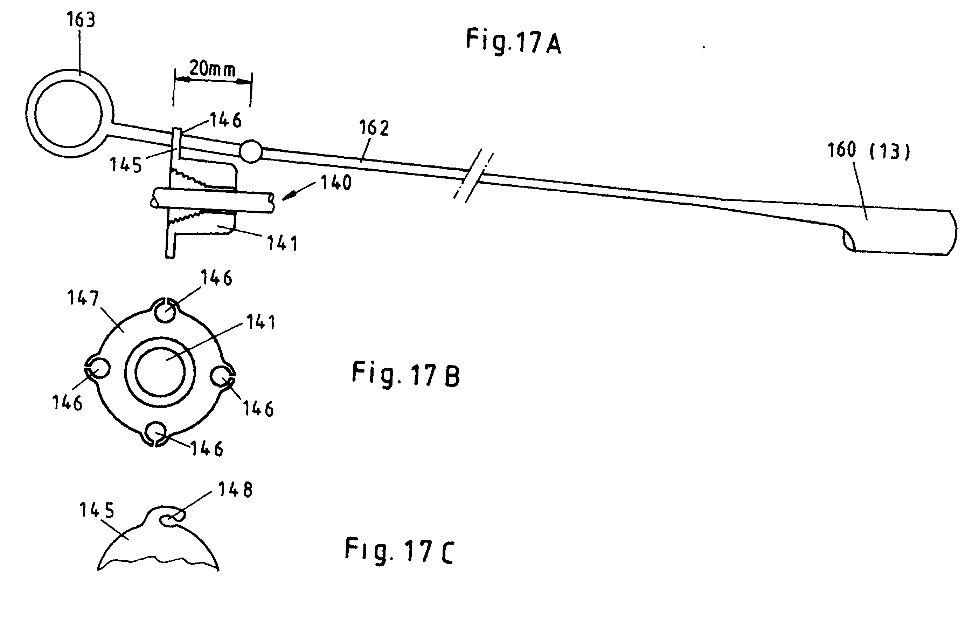

Das Überwurfrohr 13 bzw. die Aufreißhülse 160 laufen in eine stab- bzw. fadenartige Verlängerung bzw. Reißleine 162 aus, die am spritzenkörperseitigen Ende 162a in die fingerringartige Handhabe bzw. Lasche 163 übergeht, die die gleiche Wandstärke wie das Überwurfrohr bzw. die Aufreißhülse aufweist, wobei eine ausreichende Reißfestigkeit durch die Breite der Lasche 160 erreicht wird, wobei spritzenkörpernah die Lasche zunehmend dick und rund ausgebildet ist. Die Reißleine 162 ist dabei durch einen Ring 145 geführt, der an der auf das dem Spritzenkörper zugewandten Ende des Trägerrohres 140 aufgeschraubten Feststellmutter 141 hindurchgeführt ist (Fig. 17 A). Wie Fig. 17 B zeigt, kann die Feststellmutter 141 auch mit einer umlaufenden Ringscheibe 147 versehen sein, in deren Randbereich eine Anzahl von Durchbrechungen bzw. Augen 146 ausgebildet sind, die über Einführschlitze mit dem umlaufenden Rand in Verbindung stehen, damit von außen die Reißleine 162 in die Durchbrechung 146 eingelegt werden kann. Fig. 17 C zeigt eine hakender Feststellmutter 141, die ebenfalls zur Aufnahme und Führung der Reißleine 162 geeignet ist.

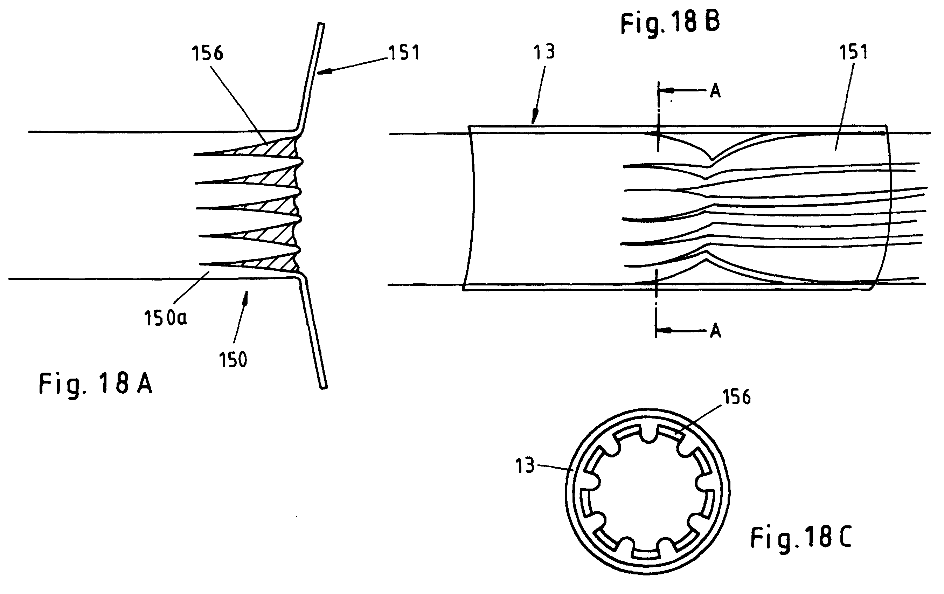

An der Basis des Faltenfächers des Faltenschirmes 150 zum Trägerrohr 140 weist nach einer weiteren Ausführungsform gemaß Fig. 18 A, Fig. 18 B und 18 C das Trägerrohr eine Art Speichenbildung auf, d. h. das Trägerrohr ist an seinem freien Ende mit einem speicherförmig ausgebildeten Abschnitt versehen, wobei zwischen je zwei Speichen eine geringe Stärke aufweisende, d. h. dünne, schwimmhautartige Folienabschnitte 156 angeordnet sind. Beim Zusammenfalten des Fächers kann dadurch die 90° Knickspannung auf eine längere Strecke ideal-elastisch eingeleitet werden, so dass beim Zusammenfalten keine bleibenden Verformungen entstehen, die die Selbstentfaltung zerstören würde.

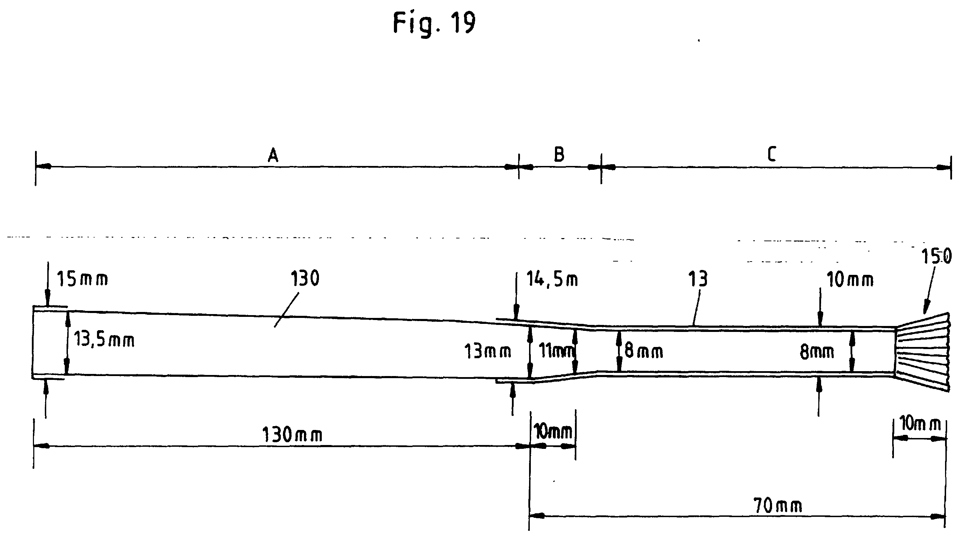

Fig. 19 zeigt die Aufteilung der Zementspritze in drei Abschnitte A, B und C. Der Abschnitt A umschließt die Spitze handelsüblicher Spritzen. Im Abschnitt B treffen die Querschnitte der handelsüblichen Spritzen auf ein konisch zulaufendes Umfangsrohr, in dem sich die Öffnung des Innenrohres bzw. des Füllrohres 130 gegen das Umfangsrohr zementdicht abschließen kann. Im Abschnitt B geht der Außendurchmesser auf kurzer Strecke auf 10 mm zurück, so dass ein für kleine Knochenhohlraumquerschnitte ausreichend schlankes Rohr erreicht wird. Der Abschnitt C verbleibt auf längerer Strecke schlank, so dass die Spitze tief in das sich verjüngende Knochenrohr 16a hineingeschoben werden kann, ohne dass die Auftreibung von Abschnitt B nach Abschnitt A sperren kann.

## Patentansprüche

1. Vorrichtung (100; 100') zum Einbringen von Knochenzement (12) vermittels einer Zementspritze (110) in ein Knochenrohr (16a) eines Knochens (16), wobei die Vorrichtung ein in das Knochenrohr (16a) einführbares Füllrohr (14) aufweist,
**dadurch gekennzeichnet,**
dass die Austrittsöffnung (15) des Füllrohres (14) einen während des Füllvorganges veränderbaren Querschnitt hat, wobei die Vorrichtung (100; 100') integrierter Bestandteil der Zementspritze (110) ist oder als flexibler Aufsatz (105) für die Zementspritze (110) ausgebildet ist.

2. Vorrichtung nach Anspruch 1,
**dadurch gekennzeichnet,**
dass die Vorrichtung eine Sperreinrichtung enthält, die im aktiven Zustand den maximalen Öffnungsquerschnitt der Austrittsöffnung (15) begrenzt.

3. Vorrichtung nach Anspruch 2,
**dadurch gekennzeichnet,**
dass die Sperreinrichtung durch ein Überwurfrohr (13) gebildet wird, das axial verschiebebeweglich koaxial zum Füllrohr (14) angeordnet ist.

4. Vorrichtung nach einem der Ansprüche 1 bis 3,
**dadurch gekennzeichnet,**
dass die Austrittsöffnung (15) des Füllrohres (14) ganz oder teilweise aus einem faltbaren, flexiblen Material (17, 19) besteht und trichterförmig zugeschnitten ist.

5. Vorrichtung nach einem der Ansprüche 1 bis 4,
**dadurch gekennzeichnet,**
dass die Austrittsöffnung (15) des Füllrohres (14) ganz oder teilweise aus sich überlappenden und jeweils einseitig an dem Füllrohr (14) befestigten Lamellen (20) besteht.

6. Vorrichtung (100; 100') zum Einbringen von Knochenzement (12) in ein Knochenrohr (16a) eines Knochens (16), enthaltend ein in das Knochenrohr (16a) einführbares Füllrohr (14),
**dadurch gekennzeichnet,**
dass die Vorrichtung eine Absaugeinrichtung (22) mit einer bei der Austrittsöffnung (15) des Füllrohres (14) angeordneten Absaugöffnung aufweist.

7. Vorrichtung nach Anspruch 6,
**dadurch gekennzeichnet,**
dass die Absaugeinrichtung (22) durch ein Überwurfrohr (13) gebildet wird, das koaxial und mit Zwischenraum zum Füllrohr (14) angeordnet ist.

8. Vorrichtung nach Anspruch 7,
**dadurch gekennzeichnet,**
dass die Absaugöffnung des Überwurfrohres (13) einen während des Füllvorganges veränderbaren Querschnitt aufweist.

9. Vorrichtung nach Anspruch 8,
**dadurch gekennzeichnet,**
dass die Absaugöffnung des Überwurfrohres (13) ganz oder teilweise aus einem faltbaren, flexiblen Material (21) besteht und trichterförmig zugeschnitten ist.

10. Vorrichtung nach einem der Ansprüche 8 oder 9,
**dadurch gekennzeichnet,**
dass die Absaugöffnung des Überwurfrohres (13) aus sich überlappenden und jeweils einseitig am Überwurfrohr (13) befestigten Lamellen besteht.

11. Vorrichtung nach einem der Ansprüche 1 bis 10,
**dadurch gekennzeichnet,**
dass sie eine Spüleinrichtung zum Einbringen von Spülflüssigkeit aufweist.

12. Vorrichtung nach einem der Ansprüche 1 bis 11,
**dadurch gekennzeichnet,**
dass die Vorrichtung (100') als flexibler Aufsatz (105) für eine Zementspritze ausgebildet ist und aus einem Füllrohr (130) mit einem einendseitig angeordneten Anschlussstutzen (131) für den Anschluss an die Zementspritze, aus einem das Füllrohr (130) aufnehmenden einendseitig konisch oder zylindrisch ausgebildeten Trägerrohr (140) mit am freien dem Spritzenkörper abgewandten Ende (140b) angeordneten Faltenschirm (150) und aus einem auf das freie Ende (140b) des Trägerrohres (140) aufgeschobenen, konischen oder zylindrischen, den Faltenschirm (150) übergreifenden Überwurfrohr (13) bzw. Aufreißhülse (160) mit einer Reißleine (162) zum Abziehen des Überwurfrohres (13) bzw. der Aufreißhülse (160) vom konischen Endabschnitt (140c) oder dem zylindrischen Endabschnitt des Trägerrohres (140) besteht.

13. Vorrichtung nach Anspruch 12,
**dadurch gekennzeichnet,**
dass das Überwurfrohr (13) als Aufreißhülse (160) ausgebildet ist.

14. Vorrichtung nach einem der Ansprüche 12 oder 13,
**dadurch gekennzeichnet,**
dass die Aufreißhülse (160) mit einem Längsschnitt (161) versehen ist und eine Eigenspannung zum Zusammenhalten des Faltenschirmes (150) in geschlossener und zusammengefalteter Stellung aufweist.

15. Vorrichtung nach einem der Ansprüche 12 bis 14,
**dadurch gekennzeichnet,**
dass das Trägerrohr (140) auf dem Füllrohr (130) vermittels einer Feststellmutter (141) klemmend gehalten ist.

16. Vorrichtung nach einem der Ansprüche 12 bis 15,
**dadurch gekennzeichnet,**
dass das Trägerrohr (140) an seinem dem Faltenschirm (150) zugekehrten Ende außenwandseitig eine schneidmesserartig ausgebildete Rücklaufsperre (142) zur Verhinderung eines ungewollten Zurückgleitens des Überwurfrohres (13) bzw. der Aufreißhülse (160) beim Einschieben in das Knochenrohr (16a) und zum Aufschlitzen bzw. Aufweiten der Aufreißhülse (160) bei kräftiger Zugeinwirkung aufweist.

17. Vorrichtung nach einem der Ansprüche 12 bis 16,
**dadurch gekennzeichnet,**
dass die ungeschlitzte Aufreißhülse (160) zum Aufreißen mittels der schneidmesserartig ausgebildeten Rücklaufsperre (142) einen in Aufreißhülsenlängsrichtung verlaufenden Sollbruchbereich (164) aufweist.

18. Vorrichtung nach einem der Ansprüche 12 bis 17,
**dadurch gekennzeichnet,**
dass das Trägerrohr (140) auf seiner Außenwandfläche einen stabförmigen Längsnocken (Feder) (143) und das Überwurfrohr (13) bzw. die Aufreißhülse (160) in ihrer Wand eine Längsnut (144) für den Eingriff der Nockenleiste (143) zur verdrehsicheren Führung und Halterung des Überwurfrohres (13) bzw. der Aufreißhülse (160) auf dem Trägerrohr (140) aufweisen.

19. Vorrichtung nach einem der Ansprüche 12 bis 18,
**dadurch gekennzeichnet,**
dass die Reißleine (162) der Aufreißhülse (160) an ihrem freien Ende (162a) mit einer fingerringartigen Handhabe bzw. Lasche (163) versehen ist.

20. Vorrichtung nach einem der Ansprüche 12 bis 19,
**dadurch gekennzeichnet,**
dass die Spitze der konischen Schirmkappe eine zylindrische Form aufweist, auf der ein zylindrischer Überwurfring (158) längsverschieblich gehalten ist, dass die einzelnen Falten (150a) des Faltenschirmes (150) außenwandseitig mit noppenartigen Verstärkungen (155) als Anschlag für den Überwurfring (158) aufweisen und dass zur optimalen Dichtung des Faltenschirmes (150) gegen den Knochen die einzelnen Falten (150a) des Faltenschirmes (150) endseitig angeschrägt, also etwa 60° gegen die Faltenlängsachse angeschnitten sind, so dass die Falten (150a) bei einem Öffnungswinkel von etwa 30° anliegen.

21. Vorrichtung nach einem der Ansprüche 12 bis 20,
**dadurch gekennzeichnet,**
dass die Aufreißhülse (160) in eine stab- bzw. fadenartige Verlängerung bzw. Reißleine (162) am spritzenkörperseitigen Ende (162a) in die fingerartige Handhabe bzw. Lasche (163) ausläuft, die die gleiche Wandstärke wie die Aufreißhülse (160) aufweist, wobei eine ausreichende Reißfestigkeit durch die Breite der Lasche (163) erreicht wird, wobei spritzenkörpernah die Lasche (163) zunehmend dick und rund ausgebildet ist, und dass die Reißleine (162) durch einen Ring (145) an einer auf das dem Spritzenkörper zugewandte Ende (140a) des Trägerrohres (140) aufgeschraubten Feststellmutter (141) hindurchgeführt ist.

22. Vorrichtung nach einem der Ansprüche 12 bis 21,
**dadurch gekennzeichnet,**
dass an der Basis des Faltenfächers des Faltenschirmes (150) zum Trägerrohr (140) dieses einen speichenförmig ausgebildeten Abschnitt (156) aufweist, wobei zwischen zwei Speichen eine geringe Stärke aufweisende, d. h. dünne, schwimmhautartige Folienabschnitte (156) angeordnet sind, so dass bei einem Zusammenfalten des Fächers des Faltenschirmes (150) die 90° Knickspannung auf eine längere Strecke ideal-elastisch eingeleitet wird, so dass beim Zusammenfalten keine bleibenden die Selbstentfaltung zerstörende Verformungen entstehen.

23. Vorrichtung nach einem der Ansprüche 12 bis 22,
**dadurch gekennzeichnet,**
dass die Zementspritze einen ersten Abschnitt (A), einen zweiten Abschnitt (B) und einen dritten Abschnitt (C) aufweist, von denen der erste Abschnitt (A) die Spitze einer handelsüblichen Zementspritze umschließt, wobei im zweiten Abschnitt (B) sich die Querschnitte der Zementspritzen bzw. deren Füllrohre (130) auf ein konisch zulaufendes Trägerrohr (140) treffen, in dem sich die Öffnung des Füllrohres (130) gegen das Trägerrohr (140) zementdicht abschließt und wobei der Außendurchmesser auf kürzerer Strecke bevorzugterweise auf 10 mm zurückgeht, so dass ein für kleine Knochenhohlraumquerschnitte ausreichend schlankes Rohr erhalten wird, und der dritte Abschnitt (C) über eine längere Strecke in schlanker Form ausgebildet, so dass die Spitze der Vorrichtung (100') tief in das sich verjüngende Knochenrohr (16a) des Knochens (16) einschiebbar ist, ohne dass dabei der einen größeren Durchmesser aufweisende zweite Abschnitt (B) zum ersten Abschnitt (A) sperren kann.

24. Vorrichtung zum Einbringen von Knochenzement in ein Knochenrohr nach einem der Ansprüche 1 bis 23.
